(19) Europäisches Patentamt
European Patent Office
Office européen des brevets

(11) **EP 4 108 247 A1**

(12) **EUROPEAN PATENT APPLICATION**

(43) Date of publication:
**28.12.2022  Bulletin 2022/52**

(21) Application number: **21181293.8**

(22) Date of filing: **23.06.2021**

(51) International Patent Classification (IPC):
**A61K 35/22** (2015.01)      **A61P 35/00** (2006.01)

(52) Cooperative Patent Classification (CPC):
**A61K 35/22; A61P 35/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ostravska univerzita**
**70103 Ostrava (CZ)**

(72) Inventors:
• **Bagó, Julio Rodriguez**
  **70200 Ostrava (CZ)**
• **Hájek, Roman**
  **63500 Brno (CZ)**

(74) Representative: **Hartvichova, Katerina**
**HARBER IP s.r.o.**
**Dukelskych hrdinu 567/52**
**170 00 Praha 7 (CZ)**

(54) **URINE PROGENITOR CELLS FOR USE IN CANCER THERAPY**

(57)    The present invention relates to urine progenitor cells for use in the treatment of cancer. In particular, the treatment of cancer includes decreasing the amount of cancer cells and/or decreasing viability of cancer cells and/or decreasing the tumor size in cancer patients and/or reducing the tumor metastasis.

EP 4 108 247 A1

**Description**

Field of Art

The present invention relates to urine progenitor cells and their use in cancer therapy.

Background Art

Cancer cell-based therapy is a growing research field that comprises the utilization of autologous or allogeneic cells to fight cancer and relieve medical conditions. The therapeutic cells' sources employed so far have been variable, going from endothelial progenitor cells (EP2801583A1) to immune cells (EP2801583A1). Recent groundbreaking results in treating different hematological malignancies with T-lymphocytes modified with antigen receptors (US9629877B2) put the cancer cell-based therapy in the spotlight, becoming a pillar in cancer treatment. Still, at the time being, it is a fledgling therapy, and therefore, there are roadblocks to surpass, such as effector toxicity, homing, tumor escape, and universal access. Currently, only the cancer cell-based therapies based on T-lymphocytes modified with antigen receptors (CAR-T cells) are on the market (Kymriah from Novartis and Yescarta from Gilead). These therapies are still very expensive.

The so-called urine progenitor cells, also known as urine-derived stem cells or urine stem cells, are a subpopulation of urine-derived cells, with stem cell properties, such as multipotential differentiation and self-renewal. They can be isolated from regular voided urine from each individual via a non-invasive, simple, and low-cost approach, generating up to 100 million cells from one single urine specimen. Another essential feature of these cells is their safety as no formation of teratomas have been reported after in vivo transfusion. So far, these cells were proposed for therapeutic use only in connection with tissue regeneration (Sato M. et al.: Front. Mol. Neurosci., 05 December 2019 https://doi.org/10.3389/fnmol.2019.00297; Pavathuparambil Abdul Manaph, N., Al-Hawwas, M., Bobrovskaya, L. et al. Urine-derived cells for human cell therapy. Stem Cell Res Ther 9, 189 (2018)).

Disclosure of the Invention

The present invention provides urine progenitor cells for use in the treatment of cancer.

More particularly, the present invention provides urine progenitor cells for use in decreasing the amount of cancer cells and/or decreasing viability of cancer cells and/or decreasing the tumor size in cancer patients and/or reducing the tumor metastasis.

In some embodiments, the present invention provides urine progenitor cells for use in targeting anti-cancer agents.

Furthermore, the present invention provides a method for treatment of cancer, comprising the step of administering (a therapeutically effective amount of) urine progenitor cells to a subject in need of such treatment.

More specifically, the present invention provides a method for decreasing the amount of cancer cells and/or decreasing viability of cancer cells and/or decreasing the tumor size in a body of a subject in need of such treatment, comprising the step of administering (a therapeutically effective amount of) urine progenitor cells to the subject.

In some embodiments, the urine progenitor cells may be used (e.g., applied to a patient) straight after their isolation and optional expansion, i.e.,,, without modifications.

In some embodiments, the urine progenitor cells may be modified before use (e.g., application to a patient), typically by insertion of genes encoding: apoptosis-inducing ligands, and/or enzymes that convert non-toxic prodrugs into cytotoxic active agents, and/or growing inhibitory proteins and/or immune stimulator cytokines. The inserted genes may be under the control of constitutive or conditional promoter.

In particular, the urine progenitor cells may be modified by insertion of a gene selected from genes encoding TNF-related apoptosis-inducing ligand (TRAIL), Cytosine Deaminase (CD), Herpes simplex virus-thymidine kinase (HSV-TK), IFN- $\beta$, and IL-15. The inserted genes may be under the control of a constitutive or conditional promoter.

The expression of these genes in the urine progenitor cells can be transiently or stably integrated into the genome. For the transient expression, non-integrative plasmids can be transfected to the urine progenitor cells using reagent-based methods such as cationic polymers, DEAE-dextran, calcium phosphate, or instruments based methods such as electroporation. For the stable integration, viral vectors such as lentivirus or retrovirus can be used, as well as non-viral approaches with integrative plasmids with transposon transposase system using reagent-based methods such as cationic polymers, DEAE-dextran, calcium phosphate, or instrument-based methods such as electroporation.

In some embodiments, the urine progenitor cells may be modified before use (e.g., application to a patient) by loading with anticancer agents, such as anticancer or antitumor drugs, and/or irradiation treatment sensitizers or agents, and/or oncolytic virus. For example, the urine progenitor cells can be loaded with nanoparticle carriers containing high concentration of insoluble chemotherapeutic agents, such as doxorubicin-containing porous silica nanorattles, by specific antibody-antigen recognitions at the cytomembrane interface.

Within the framework of the present invention, it was found that urine progenitor cells exert direct effects on cancer cells. This may be due to a strong expression of several anti-tumor proteins in the urine progenitor cells. This effect may be further increased by genetic modification by insertion of genes encoding further anti-tumor proteins or ligands. Furthermore, it was found that urine progenitor cells have a propensity to migrate towards cancer cells. At the same time, it is known that the urine progenitor cells do not form teratomas and are safe for use in medical applications, which allows their use in regenerative medicine applications. This combination of activities and properties makes them an extremely suitable candidate for cancer therapy.

It should be noted that the urine progenitor cells are effective in decreasing the amounts and viability of cancer cells of various types and origins. Therefore, they may be used in the treatment of a wide range of cancers.

In particular, the cancers to be treated may be selected from a group including, for example, breast cancer, osteosarcoma, glioblastoma, lung cancer, prostate cancer, ovarian cancer, colon cancer, pancreatic cancer, liver cancer and/or melanoma.

Urine progenitor cells (unmodified, modified by insertion of genes, or loaded with anticancer agents or oncolytic virus) may be administered to patients in compositions with pharmaceutically acceptable excipients. The compositions may be liquid (e.g., infusion solutions, injection solutions, or liquid oral dosages), or solid (e.g., tablets, capsules, or lyophilized powders).

The pharmaceutically acceptable excipients typically include solvents, such as water, saline or pH 3-8 buffers, antioxidants, preservatives, salts, binders, lubricants, fillers, glidants, antiadherents.

A preferred route of administration is intravenous infusion, local tumor administration or injection.

The term "urine progenitor cells" relates to cells isolated from human urine. These cells are also sometimes referred to as "urine-derived stem cells". The term "urine progenitor cells" is abbreviated herein as "UPC" or "UPCs".

An example of a procedure for obtaining and formulating the urine progenitor cells includes the following steps:

- collecting voided urine in a sterile bottle;
- centrifugation of the collected urine and collecting the cellular pellet;
- resuspending the cellular pellet;
- optionally modifying the cells (e.g. by insertion of a gene);
- expanding the cells to the desired amount;
- optionally loading the cells with anticancer agent(s);
- optional lyophilization or cryostorage;
- formulation into the dosage form (e.g., infusion solution, injection solution).

Urine progenitor cells may be easily obtained from urine of a mammal, preferably a human. The cells are isolated and expanded to obtain the desired amounts by common scale-up cultivation techniques for adherent cells, such as wave bioreactor with microcarriers, hollow fiber bioreactors or Hyperstack cell culture vessels. Then they may be formulated into the desired dosage forms. Such simple and effective product allows an effective, affordable, and non-invasive approach to treating various cancer types.

Compared to known cancer cell-based therapies, such as the ones that use adult mesenchymal cells or immune cells, urine progenitor cells can be straightforwardly obtained in a non-invasive approach and be easily expanded using low-cost techniques, reaching clinically relevant doses in a short time. Besides, unlike other cells used in cancer cell-based therapy such as T-lymphocytes and NK cells, urine progenitor cells can be easily manipulated to increase their antitumor properties.

Furthermore, urine progenitor cells present an inherent tumor tropism, able to deliver anti-cancer properties to tumors, increasing, in consequence, their therapeutic efficacy.

Another advantage of the technology of the invention is that unlike other cancer cell-based therapies such as with CAR-T cells, urine progenitor cells don't produce rejection or toxicity in *in vivo* experiments.

The "patient" or "subject in need of treatment" is preferably a mammal, more preferably a human.

The term "decreasing" refers to decreasing the amount of cancer cells and/or decreasing viability of cancer cells and/or decreasing the tumor size. The decrease refers to the change after treatment with UPCs with respect to the status before treatment with UPCs.

The term "reducing the tumor metastasis" refers to reduction of at least one of: size, amount, rate of tumor metastasis. The reduction refers to the change after treatment with UPCs with respect to the status before treatment with UPCs.

The invention is further disclosed and illustrated by means of examples which should not be construed as limiting. The scope of the invention is defined in the claims.

Brief description of Drawings

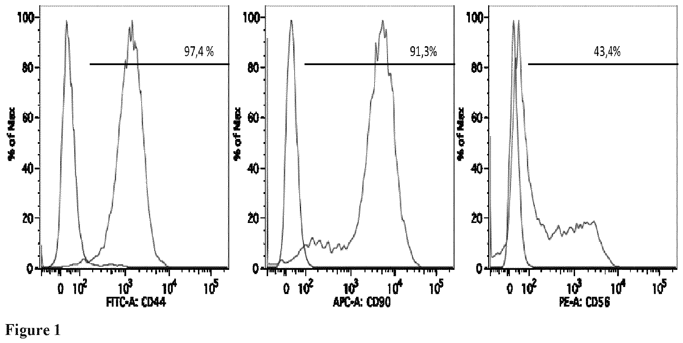

Figure 1. Phenotypic characterization of UPCs. FACS analysis with the % positive cells for CD44, CD90, and CD56 antigens in UPCs isolated from voided urine and expanded for two weeks. The data shown is a representative experiment from three different donors.

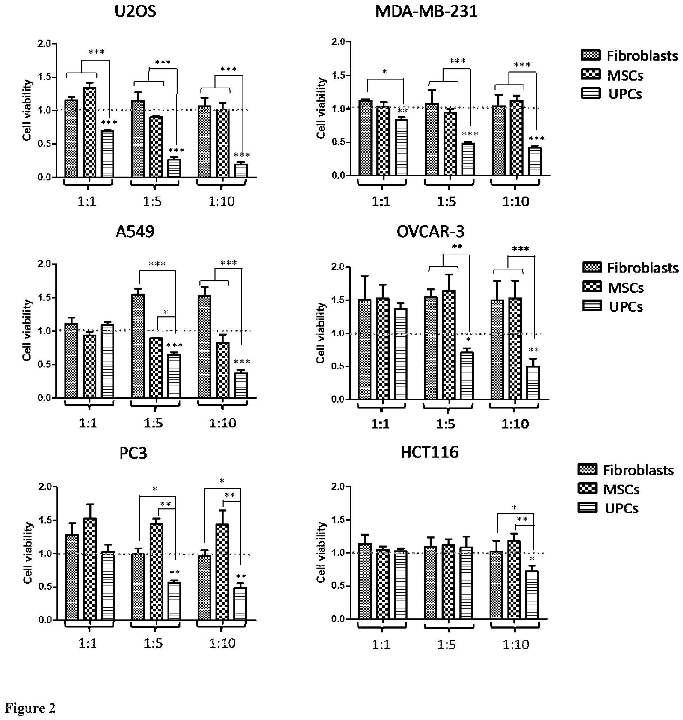

Figure 2. Graphic with the antitumor properties of UPCs against tumor cell lines MDA-MB-231, U-2OS, A549, HCT116, OVCAR-3, and PC-3 at different T:E (1:1, 1:5, and 1:10) for 72 hours. Results are compared to the ones obtained with fibroblasts and mesenchymal cells with the same conditions. Data are presented as means $\pm$ SD from three technical replicates. *, $p < 0.05$, **, $p < 0.01$, ***, $p < 0.001$, one-way ANOVA, repeated measures test.

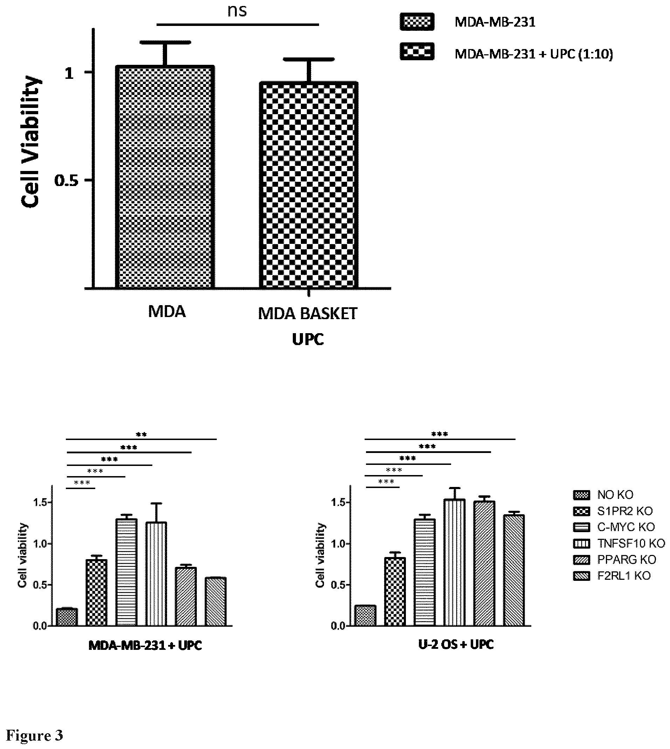

Figure 3. Cell viability of MDA-MB-231 after culture with UPCs and separated by a transwell permeable support. Data are presented as means $\pm$ SD of three technical replicates. Ns, not significant, Student's t-test Cell viability of tumor cell lines MDA-MB-231 and U2OS after coculture with the different UPCs knockouts compared to the control without knockout at a T:E of 1:10 for 72 hours. Data are presented as means $\pm$ SD from three technical replicates. *, $p < 0.05$, **, $p < 0.01$, ***, $p < 0.001$, one-way ANOVA, repeated measures test

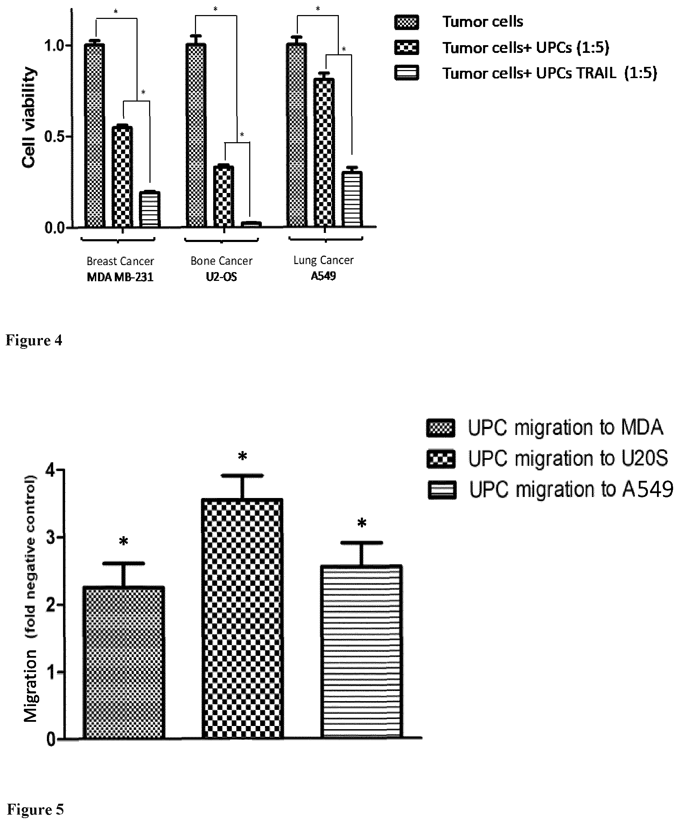

Figure 4. Anti-tumor properties of UPCs and UPCs engineered for the expression of TRAIL against the tumor cell lines MDA-MB-231, U-2OS, and A549 at a T:E of 1:5 for 72 hours. *P < 0.05 by student's t-test. Data are presented as means $\pm$ SD in three technical replicates.

Figure 5. Transwell migration assay of UPCs to assess their motility towards conditioned media from MDA MB-231, U2-OS, and A549. Bar graphs showing how the conditioned media of the different tumor cell lines significantly promoted UPCs' migration, compared to the negative control (no conditioned media). *P < 0.05 by student's t-test. Data are presented as means $\pm$ SD in three technical replicates.

Examples of carrying out the Invention

**Example 1: Isolation and characterization of urine progenitor cells (UPCs)**

The UPCs were isolated from three different donors' voided urine samples (age range 35-45). Voided urine samples (~500 ml) were centrifuged at 300xg for 5 min. The supernatant was removed, and the remaining cell pellet was washed with PBS and centrifuged at 300xg for 5 min. The supernatant was removed and the cell pellet cultivated in a 24 well plate with a 1:1 medium of defined Keratinocyte-SFM (Thermo Fisher) and complete embryonic fibroblast medium (Cell Biologics). A day later, the medium was changed to remove the detritus and unattached cells. Confluence was achieved in 2-3 weeks. The medium was changed every other day. In order to study the proliferation capacity of a single UPC, a limiting-dilution method was performed. Briefly, the UPCs were diluted in 96 cell well plates to a cell density as low as 0.5 cells per well by transferring 0.1 ml of a cell suspension with five cells/ml. According to a Poison distribution, the seeding of such an average of cells ensures that some wells will receive a single cell, minimizing the probability that any well receives more than one. Single clones proliferation were monitored over time, and duplication time was calculated with the following formula:

$$Doubling\ time = \frac{time \times \log(2)}{\log(Final\ Concentration) - \log(Initial\ Concentration)}$$

The average number of UPCs in every 200 ml of voided urine was four. Applying the limiting-dilution method, we studied the proliferation capacity of single UPC clones. Every single clone's proliferation was monitored over time, obtaining a median duplication time of 27 hours and a median of $2.03 \times 10^5$ cells per clone.

Next, we conducted the phenotypic characterization of the isolated and expanded UPCs by flow cytometry BD FACSAria™ III Cell Sorter (BD Biosciences-US)) with the following antibodies: CD44-FITC, CD90-APC, and CD56-PE. As expected, the UPCs were positive for the expression of the stem cell markers CD44 and CD90 and the expression of the neural cell adhesion marker (CD56) (Figure 1). This example indicates the feasibility in the isolation and expansion of UPCs from different donors, achieving clinically relevant cell numbers.

**Example 2: Anti-tumor activity of UPCs against cancer cell lines**

The ability of the UPCs to exert anti-tumor activity against different tumor cell lines was evaluated. To this end, a bioluminescence-based cytotoxicity assay (Karimi, M. A.; Lee, E.; Bachmann, M. H.; Salicioni, A. M.; Behrens, E. M.; Kambayashi, T.; Baldwin, C. L., Measuring cytotoxicity by bioluminescence imaging outperforms the standard chromium-51 release assay. PLoS One 2014, 9 (2), e89357) was performed based on the coculture of the expanded UPCs with different human tumor cell lines (breast cancer MDA-MB-231, osteosarcoma U-2OS, lung cancer A549, colon cancer HCT116, ovarian cancer OVCAR-3 and prostate cancer PC-3) previously labeled with the expression of Firefly Luciferase at different target (T) (tumor cells) : effector (E) (UPCs) rates for 72 hours. To stable integrate the expression of firefly luciferase in the tumor cell lines, we infected them with lentivirus that carries the lentiviral construct with the constitutive expression of the red fluorescence (mCherry; mCh), and bioluminescence (firefly luciferase; FLuc) reporters. The construct was generated by amplifying the cDNA encoding both reporters from Addgene Plasmid #44965 and cloned in a lentivirus backbone (#12262, Addgene, Watertown, MA, USA) using standard cloning procedures. The lentivirus construct was packaged as a lentivirus vector in human embryonic kidney 293FT cells as described previously (Motais B, Charvátová S, Walek Z, Hrdinka M, Smolarczyk R, Cichon T, Czapla J, Giebel S, Šimíček M, Jelinek T, Ševcíková T, Sobotka J, Korístek Z, Hajek R, Bagó JR. Selection, Expansion, and Unique Pretreatment of Allogeneic Human Natural Killer Cells with Anti-CD38 Monoclonal Antibody for Efficient Multiple Myeloma Treatment. Cells 2021, 10(5), 967). The tumor cell lines were infected with the lentivirus vector at a varying multiplicity of infection in culture media containing 8 $\mu$g/mL of Polybrene (Sigma).

Antitumor assays were performed in triplicates in flat white 96-well plates with a clear bottom (Thermo Scientific). In each well, 2000 tumor cells (target cells) labeled with luciferase expression were mixed with 2000 UPCs (T:E = 1:1), 10000 UPCs (T:E = 1:5) and 20000 (T:E = 1:10) in a final volume of 100 RPMI 1640 complete medium. Seventy-two hours after cultivation, the number of target cells was quantified by adding D-luciferin potassium salt (Goldbio) to a final concentration of 0.5 mg/ml in the wells and immediately measuring the bioluminescence with the Infinite F Plex microplate reader (Tecan).

As shown in Figure 2, a significantly lower number of tumor cells in cocultures with UPCs than the control without UPCs was observed, with progressive lower tumor cell numbers with the increase of UPCs in the coculture. The experiment was performed on three different UPCs donors with similar results in all of them.

The data obtained indicate the UPCs' antitumor capacity towards different tumor cell lines.

The same cocultivation experiment was performed, but instead of UPCs as effector cells, mesenchymal stem cells (MSCs) derived from the cord blood, and fibroblast cell line hTERT-BJ were used. The MSCs present similar characteristics to the UPCs, with a multipotent differentiation capacity and the expression of the same cell surface markers such as CD44 and CD90. No antitumor effect was observed after cocultivation of the MSCs and fibroblasts with the different tumor cell lines (Figure 2), confirming the unique antitumor capacity of the UPCs. The MSCs and fibroblasts were cocultured with the tumor cell lines MDA MB-231, U-2OS, A549, HCT116, OVCAR-3, and PC-3 at different T:E ratios. No significant reduction in tumor cells' viability was achieved after coculture with the MSCs or fibroblasts at any T:E ratio studied.

**Example 3: Study of the mechanism of action of the anti-tumor properties of the UPCs**

A further series of experiments was conducted to reveal the antitumor mechanism of action of the UPCs. First, we performed an experiment to reveal if the UPC's antitumor effect was triggered by cell-to-cell contact or by the secretion of soluble factors. To this end, we cultivated the UPCs and tumor cells (as described in Example 2) separated by a small pore size membrane (0.4 $\mu$m) that permits the diffusion of soluble factors but blocks the pass of cells, avoiding the direct contact between both populations. The UPCs were cultivated with MDA MB-231 separated by the small-sized pore membrane for 72 hours at a ratio of 1:10 (T:E). As shown in Figure 3, no significant differences in tumor cell viability were registered under these conditions, proving the need for cell-to-cell contact to obtain the antitumor effect. As it was ascertained that the UPC's antitumor response depends on cell-to-cell contact, we focused our attention on finding possible proteins expressed in the UPC's membrane capable of triggering an antitumor response towards three main known mechanisms: apoptosis, phagocytosis, and extrusion. To this end, we studied the overexpression of these proteins in publicly available gene expression datasets, where the gene expression of UPCs can be compared to MSCs and fibroblasts (Rahman, M. S. et al.: Sci Rep 2020, 10 (1), 739; Beeravolu, N. et al.: Stem Cell Res 2016, 16 (3), 696-711; Kim, K. et al.: J Clin Med 2020, 9 (3)).

From this analysis, we found the following five overexpressed proteins in the UPCs' membrane that appear to be related to the antitumor response: Tumor Necrosis Factor ligand superfamily member 10 (TNFSF10), which binds to TNFRSF10A/TRAILR1, TNFRSF10B/TRAILR2, TNFRSF10C/TRAILR3, TNFRSF10D/TRAILR4 in tumor cells and trigger their apoptosis. Coagulation Factor II receptor-like 1 (F2RL1) and the peroxisome proliferator-activated receptor gamma (PPARG), which activation enhances the phagocytosis. Transcription factor c-Myc (C-MYC) is a key protein in regulating cell competition in a cell-cell interaction mechanism capable of triggering the cell death of surrounding cells by apoptosis, phagocytosis, and apical extrusion. Sphingosine-1-phosphate receptor 2 (S1PR2) is involved in the epithelial defense against cancer by apical extrusion of tumor cells. To understand the grade of these proteins' implication in the antitumor capacity, we analyzed the antitumor ability of the UPCs towards tumor cell lines MDA-MB-231 and U20S after UPCs knockout by CRISPR/CAS9 genome editing of the genes encoding these five proteins.

For generating the knockouts, CRISPR-Cas9 technology of two vector systems was used. The plasmids for transfection were ordered from Addgene (Lenti-guide puro #52963 and lenti-cas9 blast #52962). The guide RNAs were designed using https://chopchop.cbu.uib.no/ website. Three guides RNA (sgRNA) were ordered per gene and cloned into Lenti-guide Puro vector using standard cloning procedures. The lentivirus constructs were packaged as a lentivirus vector in human embryonic kidney 293FT cells as described previously (Motais B, Charvátová S, Walek Z, Hrdinka M, Smolarczyk R, Cichon T, Czapla J, Giebel S, Šimíček M, Jelinek T, Ševcíková T, Sobotka J, Korístek Z, Hájek R, Bagó JR. Selection, Expansion, and Unique Pretreatment of Allogeneic Human Natural Killer Cells with Anti-CD38 Monoclonal Antibody for Efficient Multiple Myeloma Treatment. Cells 2021, 10(5), 967). The UPCs were infected with the different lentivirus vectors in culture media containing 8 $\mu$g/mL of Polybrene (Sigma). After 72 hours of infection, cells were selected by puromycin (1ug/ml) and blasticidin (10ug/ml) resistance. The selected cells were proceeded for experiments. A qRT-PCR was performed in all of them to assess the effectivity of the UPCs knockouts generated.

The different UPCs knockouts were coculture with tumor cell lines MDA-MB-231 and U20S at a ratio of 1:10 (T:E) for 72 hours and tumor cell numbers quantified as is described in example 2.

As shown in Figure 3, the antitumor capacity of the UPCs was compromised after knocking out the studied genes, proving the importance of these proteins as critical mediators in UPCs' antitumor response.

### Example 4: Modification of UPCs by insertion of a gene

To test the feasibility of engineering the UPCs to boost their antitumor properties, we stable integrated the constitutive expression of TRAIL in urine progenitor cells using lentivirus vectors. To this end, UPCs were infected with a lentivirus that carries the lentiviral construct with the constitutive expression of TRAIL. The gene that encodes TRAIL was obtained from Addgene (plasmid #10953) and cloned into a lentiviral backbone (#12262, Addgene) using standard cloning procedures.

The lentivirus construct was packaged as a lentivirus vector in human embryonic kidney 293FT cells as described previously (Motais B, Charvátová S, Walek Z, Hrdinka M, Smolarczyk R, Cichon T, Czapla J, Giebel S, Šimíček M, Jelínek T, Ševcíková T, Sobotka J, Korístek Z, Hajek R, Bagó JR. Selection, Expansion, and Unique Pretreatment of Allogeneic Human Natural Killer Cells with Anti-CD38 Monoclonal Antibody for Efficient Multiple Myeloma Treatment. Cells 2021, 10(5), 967). The UPCs were infected with the lentivirus vector at a varying multiplicity of infection in culture media containing 8 $\mu$g/mL of Polybrene (Sigma).

TRAIL is a member of the tumor necrosis factor superfamily and is characterized by its ability to selectively induce apoptosis in tumor cells but not in healthy cells, qualifying as a potential drug specific for various cancer types (Hingtgen, S. D.; Kasmieh, R.; van de Water, J.; Weissleder, R.; Shah, K., A novel molecule integrating therapeutic and diagnostic activities reveals multiple aspects of stem cell-based therapy. Stem Cells 2010, 28 (4), 832-41). The UPCs engineered for TRAIL expression and not engineered, were cocultured with different types of tumor cell lines (MDA MB-231, U2-OS, and A549) at a 1:5 T:E ratio. Summary graph showing cell viability of the different tumor cell lines after coculture with the UPCs for 72 hours. A significant reduction in cell viability of tumor cell lines was achieved in coculture with UPCs (see Figure 4), being considerably higher in coculture with UPCs labeled for the antitumor agent TRAIL expression. The experiment was performed on three different UPCs donors with similar results in all of them. As shown in Figure 4, UPCs expressing TRAIL can exert a solid antitumor response against all the tumor cell lines tested, increasing significantly the therapeutic effect. The data obtained prove the UPCs' capability to be engineered for increasing their inherent antitumor properties, opening the possibility to engineer them to express other antitumor agents in the future.

### Example 5: Migration assay

The homing of the UPCs to the tumor target cells is essential for an efficient antitumor response. Therefore, we performed a transwell migration assay in different-tumor conditioned media to assess the UPCs' migration capacity.

The UPCs' migration capacity was evaluated in chemotaxis transwell assays with cancer cell lines serum-free conditioned media from A549, MDA-MB-231, and U-2 OS (48 hours). Briefly, transwell permeable supports (Costar) with 8 $\mu$m pores were placed on top of a 24-well plate containing 500 $\mu$L of conditioned media from each cancer cell line. Serum-starved (24 hours) UPCs were seeded in 200 $\mu$L of medium without serum in the upper chamber, at a density of $0.5 \times 10^5$ cells/well. Twenty-four hours later, UPCs cells that migrated through the pores were collected from the bottom chamber and counted using the Luna-Stem Dual Fluorescence Cell Counter (Logos Biosystems).

Figure 5 shows the results of transwell migration assay of UPCs to assess their motility towards conditioned media from MDA MB-231, U2-OS, and A549. Bar graphs showing how the conditioned media of the different tumor cell lines significantly promoted UPCs' migration, compared to the negative control (no conditioned media). The experiment was performed on three different UPCs donors with similar results in all of them. In all three conditioned media tested, we observed a significant increase in the UPCs' migration capacity. Thus, the results presented here demonstrate the migration capacity of the UPCs towards different types of tumor cells.

**Claims**

1. Urine progenitor cells for use in the treatment of cancer.

2. Urine progenitor cells for use according to claim 1, wherein the treatment of cancer includes decreasing the amount of cancer cells and/or decreasing viability of cancer cells and/or decreasing the tumor size in cancer patients and/or reducing the tumor metastasis.

3. Urine progenitor cells for use according to claim 1, wherein the treatment of cancer includes targeted delivery of anti-cancer agents to the tumor.

4. Urine progenitor cells for use according to any one of claims 1 to 3, wherein the urine progenitor cells are modified by insertion of at least one gene encoding an apoptosis-inducing ligand, and/or an enzyme for converting non-toxic prodrugs into cytotoxic products, and/or an growing inhibitory protein, and/or an immune stimulator cytokine.

5. Urine progenitor cells for use according to any one of claims 1 to 3, wherein the urine progenitor cells are modified by insertion of at least one gene selected from genes encoding TRAIL, Cytosine Deaminase (CD), Herpes simplex virus-thymidine kinase (HSV-TK), IFN-β and IL-15.

6. Urine progenitor cells for use according to any one of claims 1 to 3, wherein the urine progenitor cells are loaded with at least one anticancer agent.

7. Urine progenitor cells for use according to claim 6, wherein the anticancer agent is selected from anticancer drugs, antitumor drugs, irradiation treatment sensitizers, irradiation treatment agents and oncolytic virus.

8. Urine progenitor cells for use according to claim 6, wherein the anticancer agent is a nanoparticle carrier containing at least one insoluble chemotherapeutic agent.

9. Urine progenitor cells for use according to any one of claims 1 to 8, wherein the cancer is selected from breast cancer, osteosarcoma, glioblastoma, lung cancer, prostate cancer, ovarian cancer, colon cancer, pancreatic cancer, liver cancer and melanoma.

10. A pharmaceutical composition comprising urine progenitor cells and at least one pharmaceutically acceptable excipient for use in treatment of cancer.

11. The pharmaceutical composition for use according to claim 10, wherein the pharmaceutically acceptable excipient is a pharmaceutically acceptable solvent, and the composition is an infusion solution or an injection solution.

12. The pharmaceutical composition for use according to claim 11, wherein the pharmaceutically acceptable solvent is selected from water, saline and pH 3-8 buffer.

Figure 1

Figure 2

Figure 3

Figure 4

Figure 5

Europäisches
Patentamt

European
Patent Office

Office européen
des brevets

# EUROPEAN SEARCH REPORT

Application Number

EP 21 18 1293

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/065239 A1 (UNIV WAKE FOREST HEALTH SCIENCES [US]; ZHANG YUANYUAN [US] ET AL.) 10 June 2010 (2010-06-10) * page 16, line 16-28, page 17, line 11-21; claims 1-39; * ----- | 1-12 | INV. A61K35/22 A61P35/00 |

TECHNICAL FIELDS
SEARCHED      (IPC)

A61K
A61P

The present search report has been drawn up for all claims

| Place of search | Date of completion of the search | Examiner |
|---|---|---|
| Munich | 26 January 2022 | Pilch, Bartosz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 21 18 1293**

This annex lists the patent family members relating to the patent documents cited in the above-mentioned European search report.
The members are as contained in the European Patent Office EDP file on
The European Patent Office is in no way liable for these particulars which are merely given for the purpose of information.

**26-01-2022**

| Patent document cited in search report | Publication date | Patent family member(s) | Publication date |
|---|---|---|---|
| WO 2010065239 A1 | 10-06-2010 | US 2010111914 A1 | 06-05-2010 |
| | | US 2018117087 A1 | 03-05-2018 |
| | | WO 2010065239 A1 | 10-06-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2801583 A1

- US 9629877 B2

### Non-patent literature cited in the description

- **SATO M. et al.** *Front. Mol. Neurosci.,* 05 December 2019, https://doi.org/10.3389/fnmol.2019.00297
- **PAVATHUPARAMBIL ABDUL MANAPH, N. ; AL-HAWWAS, M. ; BOBROVSKAYA, L. et al.** Urine-derived cells for human cell therapy. *Stem Cell Res Ther,* 2018, vol. 9, 189
- **KARIMI, M. A. ; LEE, E. ; BACHMANN, M. H. ; SALICIONI, A. M. ; BEHRENS, E. M. ; KAMBAYASHI, T. ; BALDWIN, C. L.** Measuring cytotoxicity by bioluminescence imaging outperforms the standard chromium-51 release assay. *PLoS One,* 2014, vol. 9 (2), e89357
- **MOTAIS B ; CHARVÁTOVÁ S ; WALEK Z ; HRDINKA M ; SMOLARCZYK R ; CICHON T ; CZAPLA J ; GIEBEL S ; ŠIMÍČEK M ; JELINEK T.** Selection, Expansion, and Unique Pretreatment of Allogeneic Human Natural Killer Cells with Anti-CD38 Monoclonal Antibody for Efficient Multiple Myeloma Treatment. *Cells,* 2021, vol. 10 (5), 967

- **RAHMAN, M. S. et al.** *Sci Rep,* 2020, vol. 10 (1), 739
- **BEERAVOLU, N. et al.** *Stem Cell Res,* 2016, vol. 16 (3), 696-711
- **KIM, K. et al.** *J Clin Med,* 2020, vol. 9 (3
- **MOTAIS B ; CHARVÁTOVÁ S ; WALEK Z ; HRDINKA M ; SMOLARCZYK R ; CICHON T ; CZAPLA J ; GIEBEL S ; ŠIMÍČEK M ; JELÍNEK T.** Selection, Expansion, and Unique Pretreatment of Allogeneic Human Natural Killer Cells with Anti-CD38 Monoclonal Antibody for Efficient Multiple Myeloma Treatment. *Cells,* 2021, vol. 10 (5), 967
- **HINGTGEN, S. D. ; KASMIEH, R. ; VAN DE WATER, J. ; WEISSLEDER, R. ; SHAH, K.** A novel molecule integrating therapeutic and diagnostic activities reveals multiple aspects of stem cell-based therapy. *Stem Cells,* 2010, vol. 28 (4), 832-41